# EUROPEAN PATENT APPLICATION

(11) **EP 0 881 293 A1**
(43) Date of publication of application: **02.12.1998**
(21) Application number: 97201572.1
(22) Date of filing: 28.05.1997
(51) Int. Cl.: C12N 15/52, C12P 7/62, C12N 9/00, C12P 7/42, C12N 1/21, C07C 59/01

(54) **Production of medium chain length poly-3-hydroxy alkanoates in Escherichia coli, and monomers derived therefrom**

(71) Applicant: Eidgenössische Technische Hochschule Zürich Institut für Biotechnologie, 8093 Zürich (CH)
(72) Inventor: The designation of the inventor has not yet been filed
(74) Representative: Smulders, Theodorus A.H.J., Ir.

(57) **Abstract**

Production of medium chain length poly-3-hydroxyalkanoates (mcl-PHAs) in *Escherichia coli* is reported for the first time. To produce PHA in *E*. *coli*, a PHA polymerase encoding gene (*phaC2*) of *Pseudomonas oleovorans* GPo1 was introduced into various *E*. *coli* strains and fatty acid oxidation mutants thereof. Of these, *E*. *coli fadA/B*, *E*. *coli fadRfadA* or E. *coli fadRfadB* mutants, which are defective in specific steps of the fatty acid β-oxidation pathway, allowed production of mcl-PHAs from fatty acids ranging from octanoate to octadecanoate. Maximum amounts of about 10% PHA of total cell dry weight (w/w) were found. The predominant monomers found in these PHAs were always the same, independently of the chain length of the supplied fatty acids: 3-hydroxyoctanoate for even chain length fatty acids and a combination of 3-hydroxynonanoate and 3-hydroxyheptanoate for odd chain length fatty acids. Since the monomer composition of the produced polymer was independent of the fatty acid chain length, β-oxidation occurs in these β-oxidation mutants. The polymer may be hydrolyzed to yield interesting optically active monomers and their esters.

## Description

### Field of the invention

The invention is in the field of microbial production of polymers, in particular poly-3-hydroxyalkanoates of medium chain length, using recombinant bacteria capable of producing such polymers, and production of monomers derived therefrom.

### Background of the invention

Poly-3-hydroxyalkanoates (PHAs) are accumulated as intracellular storage materials by a wide variety of bacteria [1,2]. Due to their physical and chemical properties, PHAs are potential substitutes for petrol-based plastics, with the advantage of renewability of the sources and biodegradability on disposal [3]. The properties of the polymers are dependent on the monomer composition, which is determined by the particular bacterial species and the type of carbon source utilized [4,5,39]. PHA monomers range from a compound having 3 carbon atoms, 3-hydroxypropionate, to a compound containing 14 carbon atoms, 3-hydroxytetradecanoate. In general, PHA polymers composed of short chain length monomers, such as poly-3-hydroxybutyrate (PHB), are brittle. With increasing monomer chain length, the material gets more flexible. Poly-3-hydroxyoctanoate, for example, is an elastomer [6].

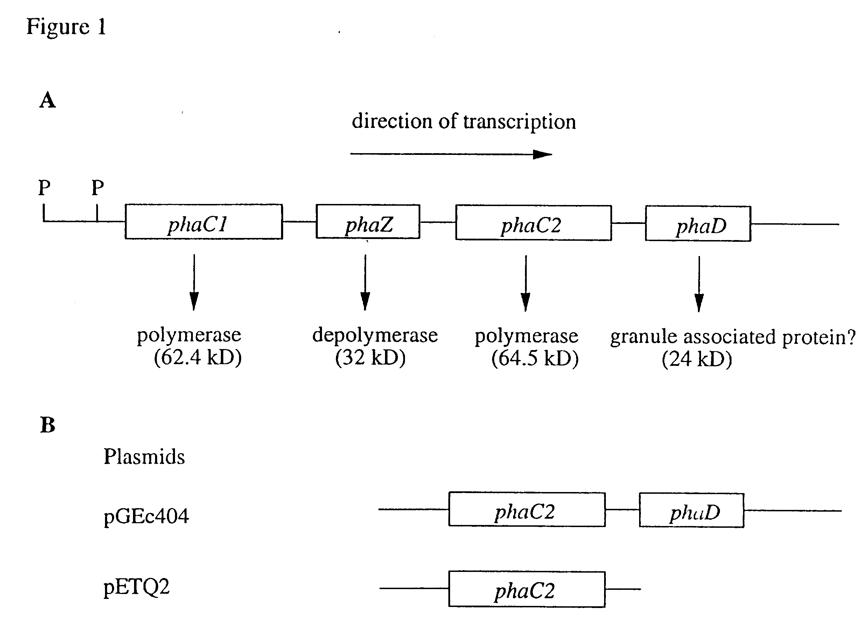

Medium chain length (mcl) PHAs (medium chain length means that they are composed of monomers containing five or more carbon atoms) were first described in *Pseudomonas oleovorans* GPol grown on n-octane [7]. Subsequently it was found that accumulation of PHAs is a common feature of fluorescent pseudomonads belonging to the rRNA homology group I [4,40]. In contrast to PHB biosynthesis, our knowledge on the genetics, physiology and regulation of mcl-PHA biosynthesis is relatively limited. A locus from *P*. *oleovorans* GPol consisting of four open reading frames (ORFs) has been cloned (Figure 1): the two ORFs encoding the polymerases are separated by an ORF that encodes a depolymerase [8]. The fourth ORF called *phaD*, may encode a granule surface protein [9]. A homologous operon from *P*. *aeruginosa* has also been identified [10]. At the amino acid level, the corresponding PHA polymerases from *P*. *oleovorans* and *P*. *aeruginosa* show 69-80% identity [8,10]. The identity between these PHA polymerases and the *A*. *eutrophus* PBB polymerase is nearly 40% [8,10].

The use of recombinant *Escherichia coli* for the production of biopolymers has several potential advantages which include fast growth, wide range of possible carbon substrates, availability of well-established high cell density culture techniques and perhaps easier and less costly downstream processing [11]. Therefore, engineering *E*. *coli* for the commercial production of mcl-PHAs may permit increased productivity and reduced product cost.

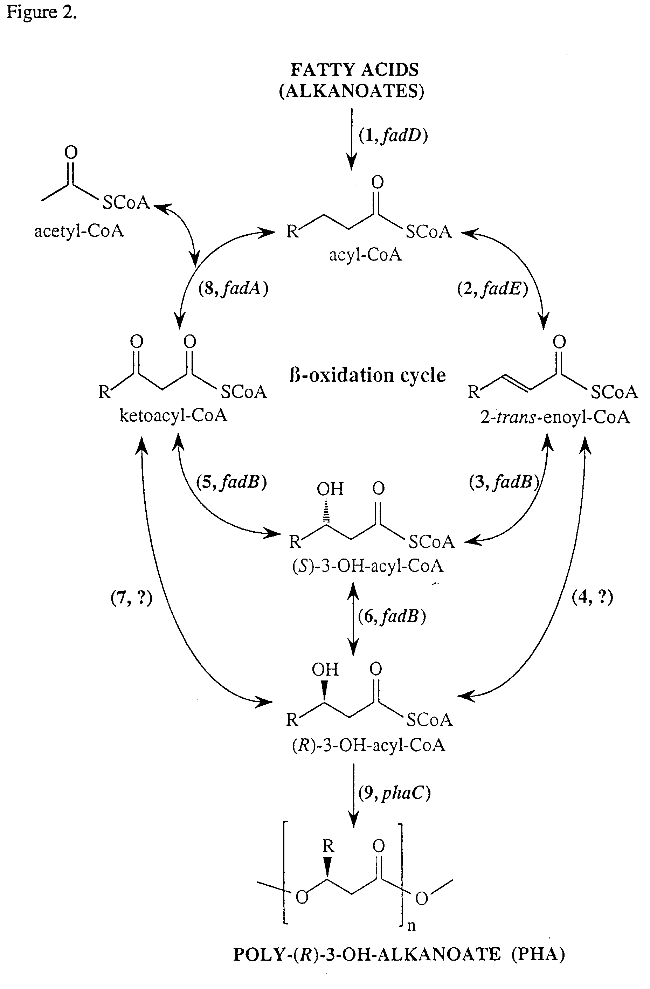

In previous studies it was hypothesized that in *Pseudomonas putida*, 3-hydroxyacyl-coenzyme A (acyl-CoA) intermediates of the β-oxidation route are channelled to PHA synthesis [9,12-14] (Figure 2). In principle, these intermediates can be accumulated also in *E*. *coli*, in which the β-oxidation pathway has been well studied, and most relevant genes have been cloned and characterized (Fig. 2, Table 1) [15-18]. Thus, expression of a PHA polymerase encoding gene in appropriate β-oxidation mutants should be sufficient to permit accumulation of mcl-PHA in *E*. *coli*.

We have investigated the possibility of PHA production in *E*. *coli* by blocking fatty acid β-oxidation to provide enough precursor for PHA polymerase. We studied the effect of *fadR*, *fadE*, *fadA/B*, *fadRfadL*, *fadRfadA* and *fadRfadB* mutations on PHA synthesis in *E*. *coli* recombinants carrying the PHA polymerase encoding gene, and report the successful production of mcl-PHAs by such engineered *E*. *coli* recombinants.

### Summary of the invention

This invention provides a process of microbial production of poly-3-hydroxyalkanoate (PHA) comprising growing a strain of *Escherichia coli* capable of producing PHA under PHA producing conditions and optionally recovering the PHA produced. More specifically, the process comprises providing a strain of *Escherichia coli* which contains a PHA polymerase and has a defect in the fatty acid β-oxidation pathway which affects fatty acid degradation but allows the synthesis of (R)-3-hydroxyacyl CoA from fatty acid, growing said strain in the presence of a fatty acid having from about 5, such as from 6, or from 7, preferably however from about 8 up to about 18 carbon atoms, or a suitable derivative of such fatty acid, and optionally recovering the PHA produced.

According to preferred embodiments, said strain of *Escherichia coli* has a *fadA* mutation affecting the *fadA* encoded 3-ketothiolase function, or a *fadB* mutation affecting the *fadB* encoded dehydrogenase function, or both mutations. The strain of *Escherichia coli* is preferably derived from an *Escherichia coli* fatty acid oxidation mutant selected from the group consisting of RS3087 (*fadA/B*), JMU193 (*fadRfadB*) and JMU194 (*fadRfadA*).

In preferred embodiments of the invention, the PHA polymerase is PHA polymerase 2 (PhaC2) from *Pseudomonas oleovorans*. However, other PHA polymerases than PhaC2 are useful as well, including e.g. PhaC1. Typically, the strain of *Escherichia coli* used harbors and expresses recombinant nucleic acid coding for said PHA polymerase. Preferably, said strain of *Escherichia coli* harbors a plasmid containing the *phaC2* gene of *Pseudomonas oleovorans*, e.g. pGEc404 or pETQ2.

According to the invention, the strain of *Escherichia coli* is grown preferably in the presence of an n-alkanoic acid having from 5, such as from 6 or from 7, preferably from 8 to 18 carbon atoms, or a mixture of such acids. Typically, the PHA produced is a medium chain length (mcl) PHA essentially composed of monomers ranging from 6 to 12 carbon atoms. Heteropolymers may contain some C4 or C5 alkanoic acid monomers, however. More specifically and preferably, the mcl-PHA produced consists of an essentially homopolymeric poly-3-hydroxyoctanoate, or consists of a copolymer essentially composed of 3-hydroxyhexanoate and 3-hydroxyoctanoate units, or of a copolymer essentially composed of 3-hydroxyoctanoate and 3-hydroxydecanoate units, or of a copolymer essentially composed of 3-hydroxyhexanoate, 3-hydroxyoctanoate and 3-hydroxydecanoate units, or of a copolymer essentially composed of 3-hydroxyheptanoate and 3-hydroxynonanoate units, or of a copolymer essentially composed of 3-hydroxyheptanoate, 3-hydroxyoctanoate and 3-hydroxynonanoate units.

This invention furthermore relates to an *Escherichia coli* strain capable of producing poly-3-hydroxyalkanoate (PHA) under PHA producing conditions. More specifically, the invention relates to an *Escherichia coli* strain which harbors recombinant expressible nucleic acid coding for a PHA polymerase and has a defect in the fatty acid β-oxidation pathway which affects fatty acid degradation but allows the synthesis of (R)-3-hydroxyacyl CoA from fatty acid.

This invention also relates to a poly-3-hydroxyalkanoate (PHA) which is obtainable (and preferably obtained) by the process according to the invention, or is obtainable (and preferably obtained) from the strain according to the invention. Furthermore, the invention relates to an optically active carboxylic acid, or ester thereof, obtainable by hydrolyzing such poly-3-hydroxyalkanoate and optionally esterifying the resulting monomers.

### Brief description of the drawings

Figure 1. Organization of the *pha* genes and plasmids carrying various *pha* constructs.
(A) The *pha* locus of *Pseudomonas oleovorans* contains at least four open reading frames (ORFs) [8]. Two polymerases and one depolymerase are encoded by the *phaCl*, the *phaC2* and *phaZ* genes, respectively. The *phaD* gene may encode a granule associated protein [9]. Possible promoters are indicated with P.
(B) pJRD215 derived plasmids containing different *pha* genes. Plasmid designations are shown in the left margin.

Figure 2. Possible links between mcl-PHA biosynthetic pathways and fatty acid metabolism.

The enzymatic activities are numbered as in Table 1. The *fadA* gene encodes 3-ketoacyl-CoA thiolase (8), the *fadB* gene encodes four enzyme activities: enoyl-CoA hydratase (3), 3-hydroxyacyl-CoA dehydrogenase (5), Δ3-cis-Δ2-trans-enoyl-CoA isomerase (not included in Fig. 2) and 3-hydroxyacyl-CoA epimerase (6). Mutations in *fadA* or *fadB* block fatty acid oxidation, and result in accumulation of specific intermediates [15-18].

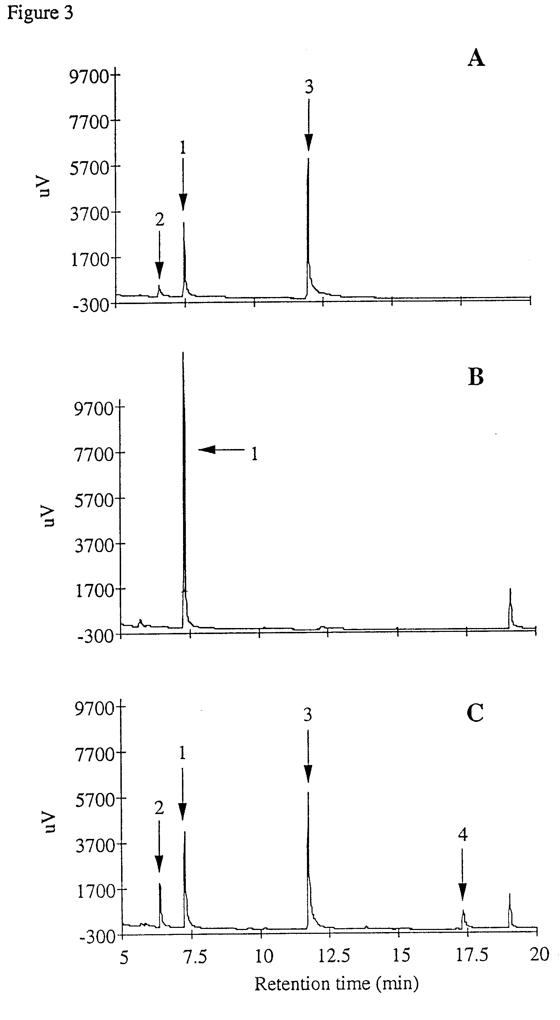

Figure 3. Gas chromatograms of methanolyzed PHA isolated from *E*. *coli* JMU194 [pGEc404].
(A) Methanolyzed pure PHA obtained from *P*. *oleovorans* GPol grown on 0.1NE2 minimal medium with octanoate as sole C-source.
(B) Methanolyzed sample from JMU194 [pGEc404] grown on 0.1NE2 with 0.2% yeast extract.
(C) Methanolyzed sample from JMU194 [pGEc404] grown on 0.1NE2 with 0.2% (w/v) yeast extract and 10 mM decanoate. Arrowhead 1, methyl benzoate (internal standard); 2, methylhydroxyhexanoate; 3, methylhydroxyoctanoate; 4, methylhydroxydecanoate. Similar results were obtained for JMU193 [pGEc404] following growth under the same conditions (data not shown).

Figure 4. Electron impact GC-MS spectrum of 3-hydroxyoctanoate methyl ester derived of PHA isolated from *E*. *coli* recombinant.

*E*. *coli* JMU194 [pGEc404] was grown on 0.1NE2 with 0.2% (w/v) yeast extract and 10 mM decanoate. The structure of the m/e = 103 fragment, which is characteristic for 3-hydroxyalkanoate methylesters, is shown inside the square.

Figure 5. *E*. *coli* recombinants containing PHA granules.

*E*. *coli* samples of JMU193 [pGEc404] (A) and JMU194 [pGEc404] (B) were stained with Sudan Black and the PHA granules visualized by phase-contrast microscopy (magnification ca. x 5,000). The *E*. *coli* recombinants were grown on 0.1NE2 with 0.2% (w/v) yeast extract and 2 mM hexadecanoate. Most of the JMU193 [pGEc404] cells contained only one PHA granule at the end of the cell and the cell shape remained normal. Most of the JMU194 [pGEc404] cells formed filaments which contained several PHA granules.

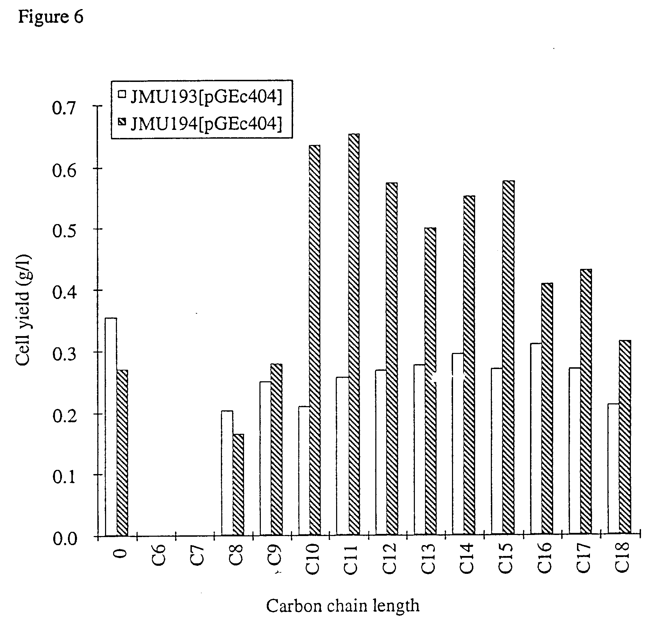

Figure 6. Growth of *E*. *coli* JMU193 [pGEc404] and JMU194 [pGEc404] on different n-alkanoates.

Cells were cultivated at 37°C for 72 h on 0.1NE2 minimal medium containing 0.2% yeast extract (w/v) and n-alkanoates with carbon chain lengths ranging from C6 to C18. The cell yield is given in g/l. Each bar represents the average of 3 experiments. The standard deviation did not exceed 0.014 g/l and the variance never exceeded 4.5%. No growth could be observed when C6 or C7 were added to the medium. Abbreviation 0: no fatty acid was added to the medium.

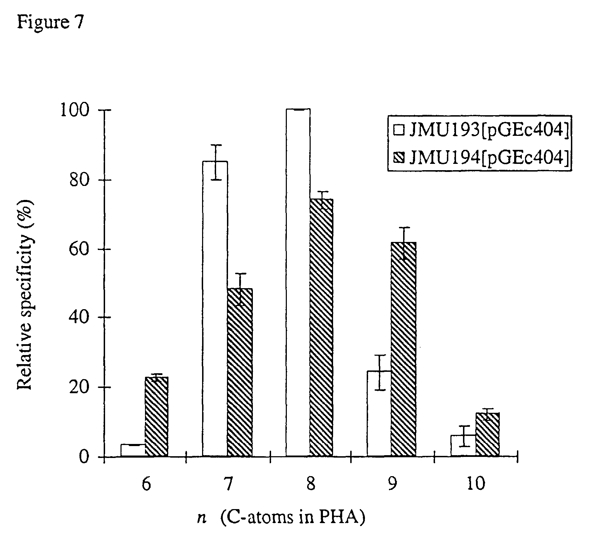

Figure 7. Frequency of monomer incorporation in PHA by *E*. *coli* recombinants.

The 3-hydroxyoctanoate monomer (C8) in the PHAs formed on C-even fatty acids in JMU193 [pEGc4O4] was taken as 100%. The other C-even monomers were calculated with respect to the C8 monomer. C-odd monomers were calculated likewise with respect to 3-hydroxyheptanoate. The ratios between C-even and C-odd monomers were calculated from PHAs formed on equimolar mixtures of tetradecanoate and pentadecanoate. Values for relative specificity are averages determined in 5-6 experiments for cells which formed PHAs on C8 to C18 fatty acids. Standard deviations are indicated.

### Detailed description of the invention

The PHA family of biological polyesters consists of several rigid PHB/V variants, and a large number of flexible mcl-PHAs [1,2]. The latter group has potentially interesting properties and applications because many monomers, of the order of 100 today, can be introduced into these bioplastics [21]. Considerable effort has been devoted in recent years to the increase of PHB yields and productivity by using genetically engineered *E*. *coli* recombinants [22,23], and PHB production on a larger scale and at low cost, comparable to that of synthetic plastics, is now increasingly likely based on the construction of PHB producing transgenic plants [24-26].

Herein we report that there are similar perspectives for mcl-PHA production. We show that mcl-PHAs can be produced in *E*. *coli* by introducing a gene (or genes) coding for one or more mcl-PHA polymerases into appropriate *E*. *coli* strains which accumulate mcl-PHA precursors. Specifically, we introduced the *phaC2* gene of *P*. *oleovorans* into *E*. *coli fadRfadA* or *E*. *coli fadRfadB* mutants, and provided the cells with alkanoates. The amount of produced PHA exceeded 10% of total cell dry weight (w/w) when cells were presented with long chain fatty acids. The produced PHAs are copolymers composed of monomers ranging from 6 to about 12 carbon atoms. These polyesters showed a constant monomer composition for each of the two recombinant strains, independent of the supplied fatty acid. 3-Hydroxyoctanoate was always the predominant monomer in C-even alkanoate grown cells; in C-odd alkanoate grown cells, 3-hydroxyheptanoate and 3-hydroxynonanoate were the predominant monomers (see Table 3). These PHA synthesis profiles in *E*. *coli* are similar to those seen in *Pseudomonas* [4,13]. Mcl-PHA synthesis in *E*. *coli* depends on accumulation of fatty acid oxidation intermediates

It has until now not been possible to produce PHAs in *E*. *coli* by only introducing *phaC* genes into a variety of *E*. *coli* hosts [27]. We have taken this to suggest that there may be additional, as yet unidentified, genes necessary for a complete mcl-PHA synthetic pathway in *E*. *coli* or that the *E*. *coli phaC* recombinants tested thus far are unable to produce PHA precursors [9,12-14]. In this study we have addressed the latter possibility.

As previously described, three main pathways could be involved in the synthesis of PHA precursors in *P*. *putida* [9,12-14,28]: fatty acid degradation, fatty acid synthesis and alkanoate elongation. Long or medium chain length fatty acids are first degraded via the β-oxidation pathway until intermediates are formed which are substrates for the PHA synthetic system [13]. This suggests that if β-oxidation (Figure 2) is blocked downstream of 3-hydroxyacyl-CoA, at step 8, the 3-ketothiolase encoded by *fadA*, or at step 5, the dehydrogenase function encoded by *fadB*, the putative precursor of PHA synthesis, 3-hydroxyacyl-CoA, will be accumulated and can then be channeled into PHA synthesis. The results of the present study clearly demonstrate that *E*. *coli* mutants defective in FadAB functions are in fact able to synthesize mcl-PHA when equipped with the PHA polymerase 2 encoding gene. PHAs are not synthesized from non-related carbon sources, such as glucose, probably because the fatty acid synthesis route does not produce sufficient intermediates for PHA polymerase.

Wild type *E*. *coli* containing *phaC2* failed to produce mcl-PHA, presumably due to insufficient amounts of the 3-hydroxyacyl-CoA substrates for the PHA polymerase. It has been speculated that in *E*. *coli* the missing step for PHA synthesis is the formation of the proper enantiomer of 3-hydroxyacyl-CoA [9,12], namely the R form (Fig. 2), or that β-oxidation might be a tightly coupled process, such that intermediates are not accumulated. The fact that *fadAB* mutants can accumulate PHA indicates that the proper substrate (R-enantiomer) is formed. Equally clearly, *fadR* mutants which are able to grow on fatty acids must produce these precursors. The fact that PHA is nevertheless not accumulated by *phaC* containing *fadR* recombinants suggests that the concentration of 3-R-hydroxyacyl-CoAs formed in these recombinants is too low for measurable PHA formation, either because the β-oxidation cycle processes these intermediates too rapidly or because intermediates are somehow physically inaccessible to the PHA polymerase.

### Fatty acid oxidation in fadA and fadB mutants

PHA polymerase shows a broad substrate specificity range [4], and in its native host, *P*. *oleovorans*, it incorporates C6 to C12 hydroxyalkanoate precursors into PHA [4]. We found the same for recombinant *E*. *coli* JMU193 and JMU194 harboring *phaC2*, either on pGEc404, or on pETQ2 (see Fig. 1). This indicates that a fully active PHA polymerase is formed in these recombinants, and that no other *pha* encoded proteins are necessary for PHA synthesis in *E*. *coli*. The fact that up to 10% PHA (w/w) was found also indicates that there is β-oxidation of fatty acids and a set of several (at least C6 to C12) 3-hydroxyacyl-CoA intermediates accumulates to levels sufficient to serve as PHA precursors by PHA polymerase. This means that exogenously supplied fatty acids must cycle through the β-oxidation system from one to six times, which is not what we had expected for these β-oxidation mutants, because it has been shown that *E*. *coli* JMU194 has no 3-ketothiolase activity [29] (FadA, step 8 in Fig. 2), while JMU193 has no 3-hydroxyacyl-CoA dehydrogenase activity [29] (FadB, step 5 in Fig. 2). However, the original activity assays, which showed no activities for these enzymes, were done with acetoacetyl-CoA (reactions 5 and 8) and crotonyl-CoA (reaction 3) as substrates [29]. Our results indicate that these *fadA* and *fadB* mutants retain some activity towards medium or long chain length fatty acid intermediates. As shorter chain intermediates are formed, these are degraded more slowly, or not at all. Thus, mcl-PHA precursors accumulate and PHA can be formed. Even if C6 and C7 intermediates might still be degraded to C4 and C5 intermediates, these will be poor precursors for mcl-PHA synthesis [4,8,9], and they will also not be degraded further to acetyl-CoA and propionyl-CoA by *fadA* or *fadB* mutants [29]. They may well accumulate to toxic concentrations, which could explain the observed toxicity of C6 and C7 fatty acids to *fadA* or *fadB* mutants. Such toxicity was not observed for *fadR* mutants, which degrade fatty acids completely and grow well on C6 or C7 fatty acids. Thus, it is to be expected that modified strains can be constructed which grow on C6 and C7 alkanoic acids and produce mcl-PHAs without accumulating shorter, toxic intermediates.

It is of course possible that there is redundancy in fatty acid oxidation, and that other enzymes complement the *fadAB* encoded functions to some extent. If so, these enzymes must have the same properties described for the mutant *fadA* and *fadB* gene products described above.

### Monomer distribution in E. coli JMU193 and JMU194

The PHA monomer compositions in the *E*. *coli fadRfadA* and *E*. *coli fadRfadB* recombinants differed significantly (Table 3a, 3b and Fig. 7). Under the same growth conditions, the *fadRfadA* recombinant incorporated more C6 and C10, and fewer C7 and C8 monomers into PHAs than the *fadRfadB* recombinant. The relative monomer incorporation rates depend on the precursor concentration and the specificity of the PHA polymerase for each precursor. The polymerizing enzyme system in *E*. *coli* recombinants obviously prefers C7, C8 and C9 monomers, while shorter or longer monomers are incorporated less efficiently (Fig. 7) as was shown to be the case for this enzyme system in *P*. *oleovorans* [4]. The enzyme specificity profile is presumably identical in *E*. *coli* recombinants JMU193 and JMU194 carrying *phaC2*. Since the monomer composition differs in these two *E*. *coli* recombinants, the relative precursor concentrations in these two *phaC2* recombinants must reflect these differences. JMU193 (*fadRfadB*) produces C8 intermediates from C16 and C18 fatty acids, and C7 intermediates from C17, but shorter (≤C15) fatty acids are degraded rather poorly. Thus, the flux of fatty acids through the β-oxidation system of this *fadB* mutant appears restricted, and C7 and C8 precursors are utilized efficiently by PHA polymerase rather than being degraded further. This may explain why there was no enhanced growth when fatty acids were supplied with yeast extract as co-carbon-source. In contrast, the fadA mutation of JMU194 allowed significant oxidation of C10 to C18 fatty acids, and a broader range of intermediates, from C6 to C9, is accumulated in sufficient concentrations to be utilized by PHA polymerase. Moreover, this recombinant not only produced more PHA, but also showed enhanced growth when fatty acids were supplied with yeast extract as co-carbon-source. It seems likely therefore that the flux of fatty acids through the β-oxidation system is strongly limited in the *fadB* mutant JMU193, and less so in the *fadA* mutant JMU194.

### Conclusions

The present report clearly demonstrates that mcl-PHA synthesis in *E*. *coli* requires only one single enzyme that is lacking in *E*. *coli*, namely PhaC, or PHA polymerase. Other proteins may enhance PHA synthesis or affect polymer granule formation [30], but none of these are essential for polymer formation. Precursors for PHA synthesis can be accumulated via native pathways in *E*. *coli*, and this requires redirecting the metabolic flux of fatty acid degradation intermediates. These results open up several interesting possibilities for future PHA production. It may be possible, by increasing *phaC* gene dosage, to increase the PHA level in recombinant *E*. *coli*, and by modifying and regulating fatty acid oxidation genes, to modify PHA compositions in engineered bacterial hosts. Redirecting various other sugar and organic acid utilization pathways towards fatty acid formation and subsequent oxidation to mcl-PHA precursors should enable us to form these plastics from a variety of carbon sources in *E*. *coli*.

### EXAMPLE 1: Investigation of PHA formation in commonly used E. coli strains

In order to investigate whether various commonly used *E*. *coli* strains (not defective in the β-oxidation pathway) were able to produce PHA when carrying a PHA polymerase encoding gene (*phaC*), pGEc404 which harbours the PHA polymerase 2 encoding gene (*phaC2*) of *P*. *oleovorans* was introduced into *E*. *coli* W3110, HB101 and DH5α, respectively. The recombinants were cultivated in minimal medium 0.1NE2 with 1% (w/v) glucose or an appropriate fatty acid (C8 to C18) as carbon source. After 72 h of growth, samples were taken and analyzed for the presence of PHA by gas chromatography (GC). No detectable (less than 0.1% (w/w) PHA per cell dry weight) PHA was formed in these recombinant *E*. *coli* strains (data not shown).

### EXAMPLE 2: PHA production in different E. coli fad mutants

Since it is likely that the precursor for medium chain length PHA synthesis is an intermediate of the fatty acid metabolism [12-14], subsequent experiments were performed with *E*. *coli* mutant strains blocked in different steps of the β-oxidation pathway: DC369 (*fadR*), K19 (*fadE*), RS3087 (*fad-751* is due to a transposon integration in the *fadAB* cluster), RS3338 (*fadRfadL*), JMU193 (*fadRfadB*) and JMU194 (*fadRfadA*). The sources of these *E*. *coli* mutants are listed in Table 2. The *fadR* gene encodes a protein that exerts negative control over genes necessary for fatty acid oxidation [15-18]. A mutation in *fadR* derepresses transcription of the genes involved in fatty acid oxidation as a result of which the *fad* genes are constitutively expressed. The *fadL* gene encodes a protein responsible for the transfer of fatty acids into the cells. A mutation in *fadL* results in a phenotype such that long chain fatty acid cannot be taken up by cells, whereas medium chain length fatty acids can diffuse through the membranes. Fig. 2 and Table 1 illustrate the functions of FadA, FadB, FadD and FadE.

The *E*. *coli* mutants were first tested to confirm their phenotype. In all cases the expected results were obtained: DC369 (*fadR*) was able to grow on minimal medium with all tested fatty acids as sole C-source; RS3338 (*fadRfadL*) was able to grow on medium chain length fatty acids (C6 to C12), but not on long chain length fatty acids (C13 to C18). K19 (*fadE*), RS3087 (*fadAB*), JMU193 (*fadRfadB*) and JMU194 (*fadRfadA*) were not able to grow on minimal medium with any of the tested fatty acids.

These *E*. *coli* fad mutants, transformed with pGEc404, were cultivated in 0.1NE2 with 10 mM decanoate. If necessary, 0.2% (w/v) yeast extract or 1% (w/v) glucose was added together with the fatty acids for growth. After 72 h, samples were taken and analyzed for PHA by gas chromatography (GC). No detectable PHA was found in E. *coli* DC369, K19 and RS3338 recombinants (data not shown). However, GC analysis of methanolyzed RS3087 [pGEc404], JMU193 [pGEc404] and JMU194 [pGEc404] samples showed a set of compounds which eluted with the same retention time as 3-hydroxyhexanoyl methyl esters and 3-hydroxyoctanoyl methyl esters (Fig. 3). These compounds were not detected when *E*. *coli* RS3087, JMU193 and JMU194 were transformed with vector pJRD215, which does not contain the *phaC2* gene, or in *phaC2*-transformed *E*. *coli* RS3087, JMU193 and JMU194 recombinants grown on 1% (w/v) glucose or 0.2% (w/v) yeast extract without fatty acids.

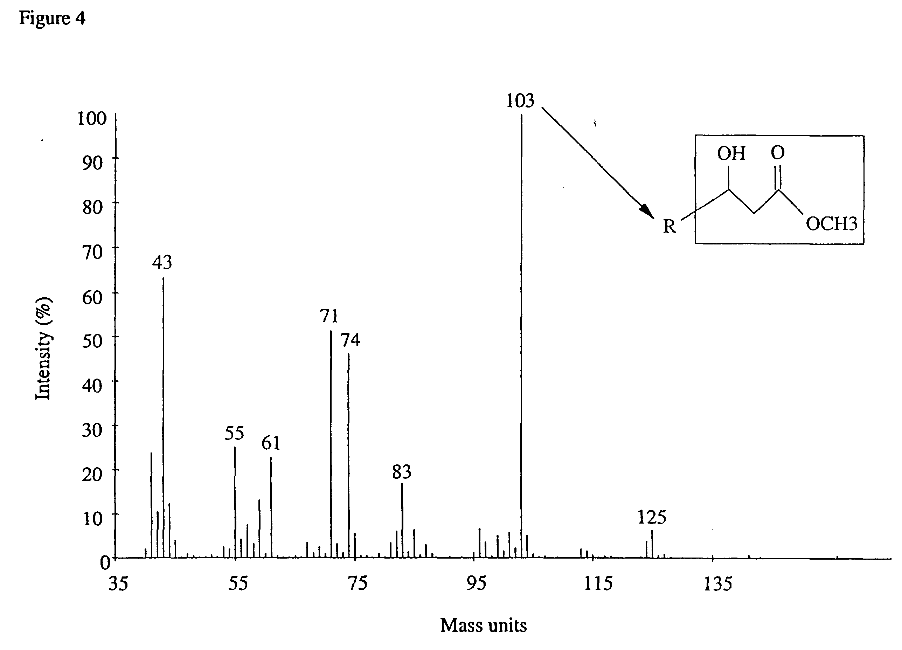

The additional compounds detected by GC were further analyzed by gas chromatography-mass spectrometry (MS). These compounds were confirmed to produce the same mass fragmentation pattern as reference samples of 3-hydroxyhexanoyl methyl esters and 3-hydroxyoctanoyl methyl esters (Fig. 4).

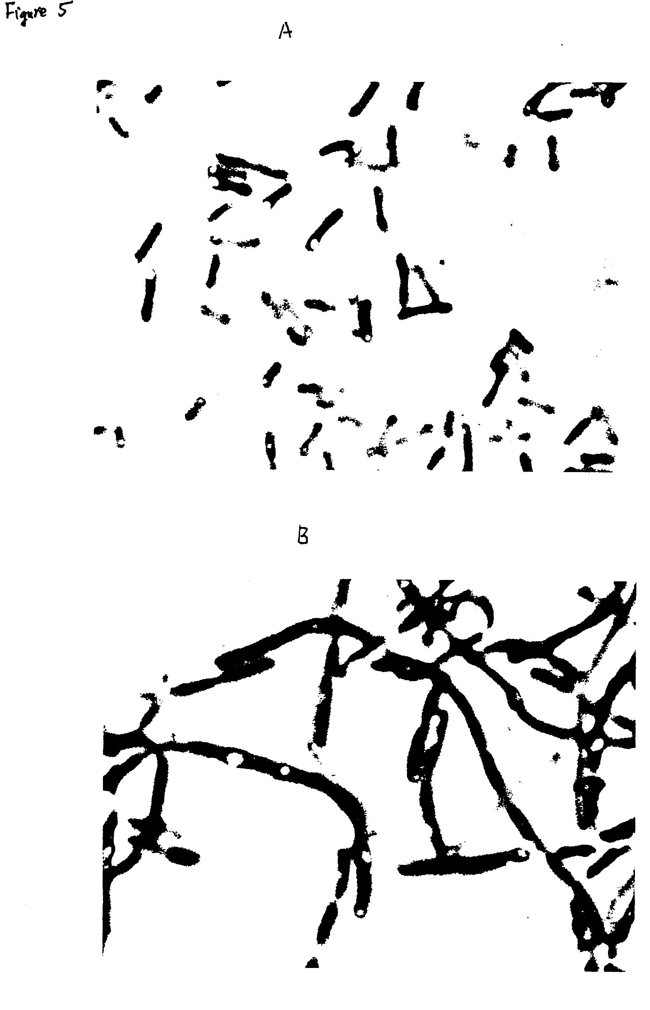

We also examined PHA producing *E*. *coli* cells with phase-contrast microscopy. Since *Pseudomonas* accumulates PHA as intracellular granules, which exhibit a shining orange color after staining with Sudan-Black [19,20], we looked for similar granules in *E*. *coli* recombinants shown to be positive for PHA production by GC-MS analysis. Shining orange granules were in fact observed in all PHA-producing samples (Fig. 5 shows black-white pictures where these granules appear bright). Similar granules were never observed in untransformed *E*. *coli* or in DC369, K19 and RS3338 *E*. *coli fad* mutants transformed with *phaC2*.

### EXAMPLE 3: Effect of different substrates on the monomer composition of the polymers produced by E. coli JMU193 and JMU194 recombinants

During cultivation on alkanoic acids or related compounds, *Pseudomonas oleovoran*s accumulates PHA copolymers, of which the monomers reflect the chain length of the carbon backbone of the substrate plus or minus one or more C2-units [13]. To determine the ability of *E*. *coli* JMU193 and JMU194 to incorporate repeating units other than 3-hydroxyoctanoate into its storage polyester, we analyzed PHA formation on a number of different fatty acids ranging from C6 to C18. Since 0.2% (w/v) yeast extract supported the growth of *E*. *coli* recombinants better than 1% glucose (w/v), and glucose is a catabolic repressor for production of fatty acid degradation enzymes, the former was used for further experiments.

*E*. *coli* JMU193 and JMU194 grew on yeast extract alone, but they did not grow on 0.2% (w/v) yeast extract in the presence of hexanoate or heptanoate (Fig. 6). This might be due to the fact that these fatty acids or their degradation intermediates are toxic to *E*. *coli fadRfadA* and *E*. *coli fadRfadB* mutants. The other alkanoates (C8-C18) had almost no effect on cell growth of JMU193 [pGEc404] compared to growth on yeast extract without alkanoates (Fig. 6). To our surprise, n-alkanoates with chain length from C10 to C18 enhanced growth of JMU194 [pGEc404] (Fig. 6), even though this strain should not be able to metabolise these fatty acids due to the *fadA* mutation. Either JMU194 is a leaky mutant and is able to degrade long chain length fatty acids (C10-C18) when yeast extract is supplied or this mutant just takes up the long chain length fatty acids (C10-C18) and accumulates them as free fatty acids in the cells.

JMU193 [pGEc404] and JMU194 [pGEc404] were grown in 0.1NE2 minimal medium with 0.2% (w/v) yeast extract supplied with fatty acids ranging from C8 to C18. Cells were harvested after 72 h, and the amount and composition of PHAs were determined. The PHA contents and the monomer compositions are shown in Table 3. The highest PHA contents (over 10% of cell dry weight) were obtained during growth in the presence of longer chain length alkanoates of C16 to C18. Interestingly, the composition of the polyesters formed from the tested alkanoates did not vary significantly with alkanoate chain length. Although β-oxidation is presumably blocked in these recombinants, the polymer produced from odd-chain length fatty acids consisted mainly of 3-hydroxyheptanoate and 3-hydroxynonanoate, while the predominant monomer units from even-chain length fatty acids are 3-hydroxyhexanoate, 3-hydroxyoctanoate and 3-hydroxydecanoate.

When odd-chain length fatty acids were used as co-carbon source, small amounts of even-chain length repeating units were found in the storage polymer. For example, when JMU193 [pGEc404] cells were fed with heptadecanoate, about 1% 3-hydroxyoctanoate (mol%) was found in the polymers, and at the same time, trace amounts of 3-hydroxyhexanoate and 3-hydroxydecanoate were observed as well.

In general, JMU194 [pGEc404] produced more PHA than JMU193 [pGEc404], especially when grown on alkanoates with chain lengths from C10 to C15. The accumulated PHA compositions in these two recombinants were also different although the dominant monomers always remained the same. The composition of the polyesters formed on C-even fatty acids was approximately 6:88:6 for the C6, C8, and C10 monomers, respectively, in JMU193 [pGEc404]; and 20:67:13 in JMU194 [pGEc404]. In the case of growth on C-odd fatty acids, the composition was 73:27 for C7 and C9 monomers, respectively, in JMU193 [pGEc404]; while almost equimolar amounts of C7 and C9 monomers were found in JMU194 [pGEc404], except for the culture grown on nonanoate which produced only 0.5% PHA and gave a ratio of about 26:74 (see Table 3 and Fig. 7).

### EXAMPLE 4: Effect of phaD on PHA production in E. coli

The construct that was used for the above experiments contains *phaD*, an ORF downstream of *phaC2* (Fig. 1). To rule out that the *phaD* gene product is involved in fatty acid oxidation or PHA production in *E*. *coli*, pETQ2 which contains only *phaC2* was constructed as described in "Materials and Methods". When grown under the same conditions as described before, *E*. *coli* recombinants JMU193 [pETQ2] and JMU194 [pETQ2] produced PHA amounts similar to those seen for JMU193 [pGEc404] and JMU194 [pGEc404] (data not shown) indicating that *phaD* is not essential for oxidation of fatty acids or synthesis of PHAs.

### MATERIALS AND METHODS

### Strains and plasmids

Strains and plasmids used in the examples described herein are listed in Table 2.

### Media and culture conditions

*E*. *coli* was grown at 37°C in complex Luria-Bertani (LB) medium [31]. To allow accumulation of intracellular PHA, 0.1NE2 minimal medium was used [32]. In some cases, 0.2% yeast extract or 1% glucose was added to the media as co-carbon source to support growth. Fatty acids were prepared and supplied in the medium as previously described [18]. Media were solidified with 1.5% (w/v) agar, and if necessary, antibiotics were added: kanamycin, 50 µg/ml; streptomycin, 100 µg/ml; ampicillin, 100 µg/ml. Cell growth was monitored by measuring optical density at 450 nm (OD450) [33].

### Recombinant DNA techniques

Isolation and analysis of plasmid DNA were carried out according to Sambrook et al [31]. Transformations of *E*. *coli* competent cells were done according to standard procedures [31]. Plasmid pETQ2 was constructed as follows: pGEc404 was digested with *Eco*RI and *Hin*dIII to get a 3.3 kb fragment containing *phaC2*. This 3.3 kb fragment was cloned into pUC18 resulting in pETQ102. PETQ102 was cut with *Hin*dIII and *Nco*I to get a 1.1 kb fragment, and another aliquot of pETQ102 was cut with *Nco*I and *Sph*I to get a 800 bp fragment. These two fragments (1.1 kb and 800 bp) were isolated and ligated into pUC18 to obtain pETQ103. pETQ103 was cut with *Eco*RI and *Hin*dIII to obtain a 1.9 kb fragment, which was cloned into pJRD215, resulting in pETQ2.

### Determination of PHA

For a qualitative analysis of the presence of PHA, colonies were examined directed on plates [32] and cells were examined by phase contrast microscopy after Sudan Black staining [19,20]. To determine the polyester content of bacteria, cells were grown in 50 ml 0.1NE2 medium supplemented with 0.2% yeast extract and the appropriate amounts of fatty acids as indicated above, and assayed for the presence and composition of PHA by gas chromatography [13]. GC-MS analyses were performed on a Fisons MD800 mass spectrometer, using electron Impact spectra (70 eV) [12].

### Reproducibility

All experiments reported herein were carried out 3 or 4 times, and averages for these independent experiments are presented. Standard deviations and variances are given in individual tables and figure legends.

**Table 1**

| Major enzymes involved in the biochemical pathways leading to formation of potential precursors of mcl-PHA, and the corresponding genes in *E*. *coli* [15-18]. | | |
|---|---|---|
| | Enzyme | Gene |
| (1) | Acyl-CoA synthetase | *fadD* |
| (2) | Acyl-CoA dehydrogenase | *fadE* |
| (3) | Enoyl-CoA hydratase | *fadB* |
| (4) | Enoyl-CoA hydratase II | ?* |
| (5) | 3-Hydroxyacyl-CoA dehydrogenase | *fadB* |
| (6) | 3-Hydroxyacyl-CoA epimerase | *fadB* |
| (7) | Ketoacyl-CoA reductase | ?* |
| (8) | 3-Ketoacyl-CoA thiolase | *fadA* |
| (9) | PHA polymerase | *phaC* |

| | | |
|---|---|---|
| * The gene encoding the enzyme is not identified. | | |

**Table 2**

| Strains and plasmids used in this study. | | |
|---|---|---|
| Strains & plasmids | Relevant characteristics | Source or reference |
| W3110 | prototroph | 34 |
| DH5α | *supE*44, Δ*lac*Ul69 (φ80*lacZ*ΔM15), *hsdR*17, *recA*l, *endA*l, *gyrA*96, *thi*-1, *relA*l | 35 |
| HB101 | *supE*44, *hsdS*20, *recA*13*, ara*-14, *proA*2, *lacY*l, *galK*2, *rpsL*20, *xyl*-5, *mtl*-l | 36 |
| DC369 | *fadR*l6, *tyrT*58(AS), *zea*-255::Tn10, *mel*-1, *butD*12 | BJB* |
| RS3338 | *fadR*60l, *fadL*771::Tn10 | BJB |
| K19 | *fadE*62, *tyrT*58(AS), *mel*-1 | BJB |
| RS3087 | *relA*1, *spoT*1,*fad*-751::Tn10, *thi*-1 | BJB |
| JMU193 | *fadR*::Tn10, *fadB*64 | 37 |
| JMU194 | *fadR*::Tn10, *fadA*30 | 37 |
| pJRD215 | RSF1010 *ori*, pl5A *ori*, Mob⁺, Km^{r}, Sm^{r} | 38 |
| pGEc404 | pJRD215, *phaC2*, *phaD*, partial *phaF*, Km^{r}, Sm^{r} | 8 |
| pETQ2 | pJRD215, *phaC2*, Km^{r}, Sm^{r} | herein |

| | | |
|---|---|---|
| * B.J. Bachmann, *E*. *coli* Genetic Stock Center, Yale University, New Haven, Conn. | | |

**Table 3a**

| Formation of PHAs by JMU193 [pGEc404] on n-alkanoates. | | | | | | |
|---|---|---|---|---|---|---|
| Substrate^{a} | 3OH FA^{b} Composition^{c} (mol % of total 30H FA)^{d} (% of cdw)^{d} | | | | | |
| | | 3OH-C6 | 3OH-C7 | 3OH-C8 | 3OH-C9 | 3OH-C10 |
| Octanoate | 0.3 (±0.1) | 0 | 0 | 100 | 0 | 0 |
| Nonanoate | 0.2 (±0.l) | 0 | 73 | 0 | 27 | 0 |
| Decanoate | 0.6 (±0.1) | 0 | 0 | 83 | 0 | 17 |
| Undecanoate | 3.8 (±0.5) | 0 | 72 | 0 | 28 | 0 |
| Dodecanoate | 4.8 (±0.5) | 7 | 0 | 89 | 0 | 4 |
| Tridecanoate | 3.0 (±0.8) | 0 | 71 | 0 | 29 | 0 |
| Tetradecanoate | 2.3 (±0.9) | 7 | 0 | 86 | 0 | 7 |
| Pentadecanoate | 2.3 (±0.7) | 0 | 97 | 0 | 3 | 0 |
| Hexadecanoate | 10.1 (±1.8) | 6 | 0 | 88 | 0 | 6 |
| Heptadecanoate | 11.3 (±1.2) | 0 | 75 | 1 | 24 | 0 |
| Octadecanoate | 8.5 (±0.7) | 7 | 0 | 88 | 0 | 5 |

| | | | | | | |
|---|---|---|---|---|---|---|
| ^{a} *E*. *coli* was grown on different fatty acids in 0.1NE2 with 0.2% yeast extract. | | | | | | |
| ^{b} The amount of 3-hydroxy fatty acids (3OH FA) is given as percentage (average ± standard deviation) of cell dry weight (cdw). | | | | | | |
| ^{c} 3OH-C6, 3-hydroxyhexanoate; 3OH-C7, 3-hydroxyheptanoate; 3OH-C8, 3-hydroxyoctanoate; 3OH-C9, 3-hydroxynonanoate; 3OH-C10, 3-hydroxydecanoate; 0, <0.1%. | | | | | | |
| ^{d} All percentages represent averages of four experiments. | | | | | | |

**Table 3b**

| Formation of PHAs by JMU194 [pGEc404] on n-alkanoates. | | | | | | |
|---|---|---|---|---|---|---|
| Substrate^{a} | 3OH FA^{b} Composition^{c} (mol % of total 3OH FA)^{d} (% of cdw)^{d} | | | | | |
| | | 3OH-C6 | 3OH-C7 | 3OH-C8 | 3OH-C9 | 3OH-C10 |
| Octanoate | 2.6 (±1.2) | 22 | 0 | 78 | 0 | 0 |
| Nonanoate | 0.5 (±0.1) | 0 | 26 | 0 | 74 | 0 |
| Decanoate, | 7.5 (±1.8) | 22 | 0 | 69 | 0 | 8 |
| Undecanoate | 5.4 (±0.7) | 0 | 45 | 0 | 55 | 0 |
| Dodecanoate | 6.0 (±1.2) | 20 | 0 | 66 | 0 | 13 |
| Tridecanoate | 6.4 (±1.2) | 0 | 50 | 0 | 50 | 0 |
| Tetradecanoate | 9.6 (±1.5) | 22 | 0 | 64 | 0 | 14 |
| Pentadecanoate | 8.4 (±1.0) | 0 | 48 | 0 | 52 | 0 |
| Hexadecanoate | 11.1 (±1.8) | 17 | 0 | 65 | 0 | 18 |
| Heptadecanoate | 8.3 (±0.9) | 0 | 50 | 0 | 50 | 0 |
| Octadecanoate | 12.3 (±0.9) | 22 | 0 | 63 | 0 | 15 |

| | | | | | | |
|---|---|---|---|---|---|---|
| ^{a} *E*. *coli* was grown on different fatty acids in 0.1NE2 with 0.2% yeast extract. | | | | | | |
| ^{b} The amount of 3-hydroxy fatty acids (3OH FA) is given as percentage (average ± standard deviation) of cell dry weight (cdw). | | | | | | |
| ^{c} 3OH-C6, 3-hydroxyhexanoate; 3OH-C7, 3-hydroxyheptanoate; 3OH-C8, 3-hydroxyoctanoate; 3OH-C9, 3-hydroxynonanoate; 3OH-C10, 3-hydroxydecanoate; 0, <0.1%. | | | | | | |
| ^{d} All percentages represent averages of four experiments. | | | | | | |

### References

1. Steinbüchel, A. 1991. Polyhydroxyalkanoic acids. 123-213 in Biomaterials. Novel Materials from Biological Sources, Byrom, D. (ed.) Macmillan Publishers Ltd, Basingstoke.
2. Anderson, A.J. and Dawes, E.A. 1990. Occurrence, metabolism, metabolic role, and industrial uses of bacterial polyhydroxyalkanoates. Microbiol. Rev. 54: 450-472.
3. Byrom, D. 1987. Polymer synthesis by microorganisms: technology and economics. Trends Biotechnol. 5: 246-250.
4. Huisman, G.W., de Leeuw, O., Eggink, G. and Witholt, B. 1989. Synthesis of polyhydroxyalkanoates is a common feature of fluorescent pseudomonads. Appl. Environ. Microbiol. 55: 1949-1954.
5. Haywood, G.W., Anderson, A.J. and Dawes, E.A. 1989. The importance of PHB-synthase substrate specificity in polyhydroxyalkanoate synthesis by *Alcaligenes eutrophus*. FEMS Microbiol. Lett. 57: 1-6.
6. Preusting, H., Nijenhuis, A. and Witholt, B. 1990. Physical characteristics of poly(3-hydroxyalkanoates) and poly(3-hydroxyalkenoates) produced by *Pseudomonas oleovorans* grown on aliphatic hydrocarbons. Macromol. 23: 4220-4224.
7. de Smet, M.J., Eggink, G., Witholt, B., Kingma, J. and Wynberg, H. 1983. Characterization of intracellular inclusions formed by *Pseudomonas oleovorans* during growth on octane. J. Bacteriol. 154: 870-878.
8. Huisman, G.W., Wonink, E., Meima, R., Kazemier, B., Terpstra, P. and Witholt, B. 1991. Metabolism of poly(3-hydroxyalkanoates) (PHAs) by *Pseudomonas oleovorans*. J. Biol. Chem. 266: 2191-2198.
9. van der Leij, F.R. and Witholt, B. 1995. Strategies for the sustainable production of new biodegradable polyesters in plants: a review. Can. J. Microbiol. 41: 222-238.
10. Timm, A. and Steinbüchel, A. 1992. Cloning and molecular analysis of the poly(3-hydroxyalkanoic acid) gene locus of *Pseudomonas aeruginosa* PAO1. Eur. J. Biochem. 209: 15-30.
11. Lee, S.Y. 1997. *E*. *coli* moves into the plastic age. Nature Biotechnology 15: 17-18.
12. Huijberts, G.N.M., de Rijk, T.C., de Waard, P. and Eggink, G. 1994. ¹³C Nuclear magnetic resonance studies of *Pseudomonas* putida fatty acid metabolic routes involved in poly(3-hydroxyalkanoate) synthesis. J. Bacteriol. 176: 1661-1666.
13. Lageveen, R.G., Huisman, O.W., Preusting, H., Ketelaar, P., Eggink, G and Witholt, B. 1988. Formation of polyesters by *Pseudomonas oleovorans*: effect of substrates on formation and composition of poly-(R)-3-hydroxyalkanoates and poly-(R)-3-hydroxyalkenoates. Appl. Environ. Microbiol. 54: 2924-2932.
14. de Waard, P., van der Wal, H., Huijberts, G.N.M. and Eggink, G. 1993. Heteronuclear NMR analysis of unsaturated fatty acids in poly(3-hydroxyalkanoates). J. Biol. Chem. 1: 157-163.
15. DiRusso, C.C. and Nunn, W.D. 1985. Cloning and characterization of a gene (*fadR*) involved in regulation of fatty acid metabolism in *Escherichia coli*. J. Bacteriol. 161: 583-588.
16. DiRusso, C.C. and Black, P.N. 1994. Molecular and biochemical analyses of fatty acid transport, metabolism, and gene regulation in *Escherichia coli*. Biochim. Biophys. Acta 1210: 123-145.
17. Nunn, W.D. 1986. Two-carbon compounds and fatty acids as carbon sources. 285-301 in *Escherichia coli* and *Salmonella typhimurium*, Neidhardt, F.C. (ed.) American Society for Microbiology, Washington, D.C.
18. Jenkins, L.J., and Nunn, W.D. 1987. Genetic and molecular characterization of the genes involved in short-chain fatty acid degradation in *Escherichia coli*: the *ato* system. J. Bacteriol. 169: 42-52.
19. Schaad, N.W. 1988. Laboratory Guide for Identification of Plant Pathogenic Bacteria. American Phytopathological Society, St. Paul.
20. Fahy, P.C. and Persley, G.J. 1983. Plant Bacterial Diseases: A Diagnostic Guide. Academic Press, New York.
21. Steinbüchel, A. and Valentin, H.E. 1995. Diversity of bacterial polyhydroxyalkanoic acids. FEMS Microbiol. Lett. 128: 219-228.
22. Fidler, S. and Dennis, D. 1992. Polyhydroxyalkanoate production in recombinant *Escherichia coli*. FEMS-Microbiol. Rev. 103: 231-236.
23. Lee, S.Y. 1996. Plastic bacteria? Progress and prospects for polyhydroxyalkanoate production in bacteria. Trends Biotechnol. 14: 431-438.
24. William, S.F. and Peoples, O.P. 1996. Biodegradable plastics from plants. Chemtech. 26: 38-44.
25. Poirier, Y., Nawrath, C., and Somerville, C. 1995. Production of polyhydroxyalkanoates, a family of biodegradable plastics and elastomers, in bacteria and plants. Bio/Technology 13: 142-150.
26. Nawrath, C., Poirier, Y. and Somerville, C. 1994. Targeting of the polyhydroxybutyrate biosynthetic pathway to the plastids of *Arabidopsis thaliana* results in high levels of polymer accumulation. Proc. Natl. Acad. Sci. USA 91: 12760-12764.
27. Huisman, G.W. 1991. Poly(3-hydroxyalkanoates) from *Pseudomonas putida*: from DNA to Plastic. Ph.D. thesis, Groningen University, The Netherlands.
28. Huijberts, G.N.M., Eggink, G., de Waard, P., Huisman, G.W. and Witholt, B. 1992. *Pseudomonas putida* KT2442 cultivated on glucose accumulates poly(3-hydroxyalkanoates) consisting of saturated and unsaturated monomers. Appl. Environ. Microbiol. 58: 536-544.
29. Spratt, S.K., Black, P.N., Ragozzino, M.M. and Nunn, W.D. 1984. Cloning, mapping, and expression of genes involved in the fatty acid-degradative multienzyme complex of *Escherichia coli*. J. Bacteriol. 158: 535-542.
30. Pieper-Fürst, U., Madkour, M.H., Mayer, F. and Steinbüchel, A. 1995. Identification of the region of a 14-kilodalton protein of *Rhodococcus ruber* that is responsible for the binding of this phasin to polyhydroxyalkanoic acid granules. J. Bacteriol. 176: 2513-2523.
31. Sambrook, J., Fritsch, E.F. and Maniatis, T. 1989. Molecular Cloning: A Laboratory Manual. Cold Spring Harbor Laboratory Press, Cold Spring Harbor, New York.
32. Huisman, O.W., Wonink, E., de Koning, G.J.M., Preusting, H. and Witholt, B. 1992. Synthesis of poly (3-hydroxyalkanoates) by mutant and recombinant *Pseudomonas* strains. Appl. Microbiol. Biotechnol. 38: 1-5.
33. Witholt, B. 1972. Method for isolating mutants overproducing nicotinamide adenine dinucleotide and its precursors. J. Bacteriol. 109: 350-364.
34. Bachmann, B.J. 1987. Derivations and genotypes of some mutant derivatives of *Escherichia col*i K-12. 1191-1219 in *Escherichia coli* and *Salmonella typhimurium* Neidhardt, F.C. (ed.) American Society for Microbiology, Washington, D.C.
35. Hanahan, D. 1983. Studies on transformation of *Escherichia coli* with plasmids. J. Mol. Biol. 166: 557-580.
36. Boyer, H.W. and Roulland-Dussoix, D. 1969. A complementation analysis of the restriction and modification of DNA in *Escherichia coli*. J. Mol. Biol. 41: 459-472.
37. Rhie, H.G. and Dennis, D. 1995. Role of *fadR* and *atoC* (Con) mutations in poly(3-hydroxybutyrate-co-3-hydroxyvalerate) synthesis in recombinant *pha*^{*+*} *Escherichia coli*. Appl. Environ. Microbiol. 61: 2487-2492.
38. Davison, J., Heusterpreute, M., Chevalier, N., Ha-Thi, V. and Brunel, F. 1987. Vectors with restriction site banks, V. pJRD215, a wide host range cosmid vector with multiple cloning sites. Gene 51: 275-280.
39. Witholt, B. and Lageveen, R.G., European patent No. 0 274 151.
40. Witholt, B., Eggink, G. and Huisman, G.W., European patent No. 0 465 546.

## Claims

1. A process of microbial production of poly-3-hydroxyalkanoate (PHA) comprising growing a strain of *Escherichia coli* capable of producing PHA under PHA producing conditions and optionally recovering the PHA produced.

2. A process according to claim 1, comprising providing a strain of *Escherichia coli* which contains a PHA polymerase and has a defect in the fatty acid β-oxidation pathway which affects fatty acid degradation but allows the synthesis of (R)-3-hydroxyacyl CoA from fatty acid, growing said strain in the presence of a fatty acid having from about 5 up to about 18 carbon atoms, preferably from 6 to 18, or from 7 to 18, most preferably from 8 to 18 carbon atoms, or a suitable derivative of such fatty acid, and optionally recovering the PHA produced.

3. A process according to claim 1, wherein said strain of *Escherichia coli* has a *fadA* mutation affecting the *fadA* encoded 3-ketothiolase function, or a *fadB* mutation affecting the *fadB* encoded dehydrogenase function, or both mutations.

4. A process according to claim 1, wherein said strain of *Escherichia coli* is derived from an *Escherichia coli* fatty acid oxidation mutant selected from the group consisting of RS3087 (*fadA*/*B*), JMU193 (*fadRfadB*) and JMU194 (*fadRfadA*).

5. A process according to claim 1, wherein said PHA polymerase is a PHA polymerase from *Pseudomonas oleovorans*, such as PHA polymerase 1 (PhaC1) or, preferably, PHA polymerase 2 (PhaC2) from *Pseudomonas oleovorans*.

6. A process according to claim 1, wherein said strain of *Escherichia coli* harbors and expresses recombinant nucleic acid coding for said PHA polymerase.

7. A process according to claim 1, wherein said strain of *Escherichia coli* harbors a plasmid containing the *phaC2* gene of *Pseudomonas oleovorans*, e.g. pGEc404 or pETQ2.

8. A process according to claim 1, wherein said strain of *Escherichia col*i is grown in the presence of an n-alkanoic acid having from 5 to 18 carbon atoms, preferably from 6 or 7 to 18 and most preferably from 8 to 18 carbon atoms, or a mixture of such acids.

9. A process according to claim 1, wherein the PHA produced is a medium chain length (mcl) PHA essentially composed of monomers ranging from 6 to 12 carbon atoms.

10. A process according to claim 1, wherein the mcl-PHA produced consists of an essentially homopolymeric poly-3-hydroxyoctanoate, or consists of a copolymer essentially composed of 3-hydroxyhexanoate and 3-hydroxyoctanoate units, or of a copolymer essentially composed of 3-hydroxyoctanoate and 3-hydroxydecanoate units, or of a copolymer essentially composed of 3-hydroxyhexanoate, 3-hydroxyoctanoate and 3-hydroxydecanoate units, or of a copolymer essentially composed of 3-hydroxyheptanoate and 3-hydroxynonanoate units, or of a copolymer essentially composed of 3-hydroxyheptanoate, 3-hydroxyoctanoate and 3-hydroxynonanoate units.

11. An *Escherichia coli* strain capable of producing poly-3-hydroxyalkanoate (PHA) under PHA producing conditions.

12. An *Escherichia coli* strain according to claim 11 which harbors recombinant expressible nucleic acid coding for a PHA polymerase and has a defect in the fatty acid β-oxidation pathway which affects fatty acid degradation but allows the synthesis of (R)-3-hydroxyacyl CoA from fatty acid.

13. An *Escherichia coli* strain according to claim 11, which strain has a *fadA* mutation affecting the *fadA* encoded 3-ketothiolase function, or a *fadB* mutation affecting the *fadB* encoded dehydrogenase function, or both mutations.

14. An *Escherichia coli* strain according to claim 11, which strain is derived from an *Escherichia coli* fatty acid oxidation mutant selected from the group consisting of RS3087 (*fadA*/*B*), JMU193 (*fadRfadB*) and JMU194 (*fadRfadA*).

15. An *Escherichia coli* strain according to claim 11, which strain harbors recombinant nucleic acid coding for a PHA polymerase from *Pseudomonas oleovorans*, such as PHA polymerase 1 (PhaC1) or, preferably, PHA polymerase 2 (PhaC2) from *Pseudomonas oleovorans*.

16. An *Escherichia coli* strain according to claim 11, which strain harbors a plasmid containing the *phaC2* gene of *Pseudomonas oleovorans*, e.g. pGEc404 or pETQ2.

17. A poly-3-hydroxyalkanoate (PHA), obtainable by the process according to claim 1 or from the strain according to claim 11.

18. An optically active carboxylic acid, or ester thereof, obtainable by hydrolyzing a poly-3-hydroxyalkanoate according to claim 17 and optionally esterifying the resulting monomers.
